(19) **Europäisches Patentamt / European Patent Office / Office européen des brevets**

(11) **EP 4 656 215 A1**

(12) **EUROPEAN PATENT APPLICATION**
published in accordance with Art. 153(4) EPC

(43) Date of publication:
**03.12.2025 Bulletin 2025/49**

(21) Application number: **24747370.5**

(22) Date of filing: **26.01.2024**

(51) International Patent Classification (IPC):
**A61M 1/36** $^{(2006.01)}$

(52) Cooperative Patent Classification (CPC):
**A61M 1/36**

(86) International application number:
**PCT/JP2024/002358**

(87) International publication number:
**WO 2024/158046 (02.08.2024 Gazette 2024/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.01.2023 JP 2023010786**

(71) Applicant: **Toray Industries, Inc.**
**Tokyo 103-8666 (JP)**

(72) Inventors:
• **SHIMADA Kaoru**
  **Otsu-shi, Shiga 520-8558 (JP)**
• **NAKANISHI Megumi**
  **Otsu-shi, Shiga 520-8558 (JP)**
• **SEKIYA Yumiko**
  **Otsu-shi, Shiga 520-8558 (JP)**
• **TAKAHASHI Hiroshi**
  **Otsu-shi, Shiga 520-8558 (JP)**
• **KIDO Mitsuko**
  **Otsu-shi, Shiga 520-8558 (JP)**

(74) Representative: **Kador & Partner Part mbB**
**Corneliusstraße 15**
**80469 München (DE)**

(54) **WATER-INSOLUBLE CARRIER FOR ACTIVATED PLATELET REMOVAL**

(57) The present invention aims to provide a water-insoluble carrier which removes activated platelets. The present invention provides a water-insoluble carrier which removes activated platelets, the carrier having a surface with a kurtosis of 0.1 to 16.0.

Fig.1

EP 4 656 215 A1

**Description**

TECHNICAL FIELD

The present invention relates to a water-insoluble carrier which removes activated platelets.

BACKGROUND ART

Thrombi are involved in the progression of pathological conditions in arteriosclerosis, cancers, infections, kidney diseases, and the like, and activated platelets are involved in thrombus formation. In view of this, attempts have been made to suppress thrombus formation by administration of an antiplatelet drug or the like. However, since antiplatelet drugs suppress the functions of not only activated platelets, but also non-activated platelets, there is a concern about side effects such as internal bleeding of the skin, nosebleed, or bleeding from the gums and the like. In particular, in cases of severe bleeding, transfusion of a platelet preparation may be required, and hence means of selectively removing activated platelets has been demanded. Further, growth factors released from activated platelets are also involved in the progression of pathological conditions in arteriosclerosis, cancers, infections, kidney diseases, and the like. In particular, platelet-derived growth factor (PDGF) and fibroblast growth factor (FGF) are known to be involved in the tumor growth in cancers, and vascular endothelial growth factor (VEGF) is known to be involved also in the progression of infections and the like. Therefore, means to remove these growth factors to inhibit the progression of the disease conditions has also been demanded.

Patent Document 1 discloses a material that is a water-insoluble carrier to the surface of which carrier a compound(s) having a charged functional group(s) is(are) bound, wherein the extending length ratio of the surface is set to 4 to 7 to allow the removal utilizing phagocytosis of an activated leukocyte-activated platelet complex, and also discloses the fact that this material can be used for treatment of inflammatory diseases such as respiratory diseases.

Patent Document 2 discloses that a protein adsorption material in which generation of microparticles is suppressed by specifying the cross-sectional shape of a sea-island composite fiber can be used for adsorption of latent transforming growth factor-$\beta$.

Patent Document 3 discloses an adsorption material in which the amount of amino groups and the number of carbon atoms in a primary aliphatic amine or secondary aliphatic amine are specified to enable adsorption of immunosuppressive proteins and suppression of platelet adsorption, and to enable application of the material to treatment of cancers.

Patent document 4 discloses an adsorption material in which the structure, the number of carbon atoms, and the arithmetic average surface roughness of a polyamine are specified to enable adsorption of immunosuppressive leukocytes and suppression of platelet adsorption, and to enable application of the material to treatment of cancers.

PRIOR ART DOCUMENTS

Patent Documents

Patent Document 1: WO 2018/225764
Patent Document 2: WO 2019/045031
Patent Document 3: WO 2019/049961
Patent Document 4: WO 2019/049962

SUMMARY OF INVENTION

PROBLEM TO BE SOLVED BY THE INVENTION

However, the target of removal by the adsorption material described in Patent Document 1 is an activated leukocyte-activated platelet complex, and the document does not mention a technique in relation to removal of activated platelets alone at all.

The adsorption material described in Patent Document 2 is a protein adsorption material in which generation of microparticles is suppressed, and the document does not mention a technique in relation to removal of activated platelets at all.

The adsorption materials described in Patent Documents 3 and 4 aim at adsorption of immunosuppressive proteins or immunosuppressive leukocytes, and are materials that suppress platelet adsorption as opposed to the present application. The document does not mention a technique in relation to removal of activated platelets at all.

In view of this, an object of the present invention is to provide an insoluble carrier which removes activated platelets.

MEANS FOR SOLVING THE PROBLEMS

The present invention has the following constitution for solving the above problems.

(1) A water-insoluble carrier which removes activated platelets, the carrier having a surface with a kurtosis of 0.1 to 16.
(2) The water-insoluble carrier according to (1), wherein the surface has a charge.
(3) The water-insoluble carrier according to (1) or (2), wherein the charge amount of the surface is 0.3 to 3.0 mmol per 1 g dry mass.
(4) The water-insoluble carrier according to any one of (1) to (3), comprising an amino group on the surface.
(5) The water-insoluble carrier according to any one of (1) to (4), which is a fiber or a particle.
(6) The water-insoluble carrier according to (5), having a diameter of 1 to 100 μm.
(7) The water-insoluble carrier according to any one of (1) to (6), comprising a polymer selected from the group consisting of polystyrene, polypropylene, polysulfone, polyether sulfone, and cellulose.
(8) The water-insoluble carrier according to any one of (1) to (7), which removes a platelet-derived growth factor, vascular endothelial growth factor, and/or fibroblast growth factor.
(9) A blood purification device, comprising the water-insoluble carrier according to any one of (1) to (8).
(10) The blood purification device according to (9), which is a blood purification column.
(11) The blood purification device according to (9) or (10), which is for suppression of thrombus formation.

EFFECT OF THE INVENTION

The water-insoluble carrier, the blood purification device, and the blood purification column of the present invention enable efficient removal of activated platelets.

BRIEF DESCRIPTION OF DRAWINGS

Fig. 1 is a schematic diagram showing one example of an image used for analysis of the kurtosis.
Fig. 2 is a schematic diagram showing the platelet fraction of CD41-positive cells in flow cytometry.

MODE FOR CARRYING OUT THE INVENTION

The present invention is a water-insoluble carrier which removes activated platelets, the carrier having a surface with a kurtosis of 0.5 to 16.0.

The "water-insoluble carrier" means a carrier that is insoluble in water. Being insoluble in water herein means that the change of dry mass of the carrier between before and after the carrier is put in water is 1% by mass or less. The dry mass change is a ratio of the dry mass of remained solid contents with respect to the dry mass of the carrier before immersed in water, which dry mass of remained solid contents is obtained by immersing the carrier, for one hour, in 37°C water whose amount is nine times larger than the dry mass of the carrier, then pulling the carrier out using tweezers or the like, and drying remained water in vacuum at 50°C or less to constant mass. In cases where the carrier is not water-insoluble, it has a risk of increasing the amount of eluate when actually used, which is not preferable from a safety point of view.

The material of the water-insoluble carrier of the present invention is not limited as long as the strength of the water-insoluble carrier can be maintained. However, from the viewpoint of maintaining the strength, and ease of processing of the surface shape of the water-insoluble carrier, examples of the material include synthetic polymers such as polystyrene, polypropylene, polyethylene, polysulfone, polyether sulfone, polyester, nylon, and polyvinyl alcohol; natural polymers such as cellulose, collagen, chitin, chitosan, and dextran; and modified natural polymers such as cellulose esters including cellulose triacetate. These polymers may be homopolymers or copolymers, or may be used as a mixture or an alloy of two or more kinds of polymers.

In particular, for use in blood purification, the material preferably contains one or more polymers selected from the group consisting of polystyrene, polypropylene, polysulfone, polyether sulfone, and cellulose from the viewpoint of the fact that their biocompatibilities have been demonstrated. Among them, the material including polystyrene is particularly preferable because it has a large number of aromatic rings per unit mass, and various functional groups or reactive functional groups are introduced easily through Friedel-Crafts reaction or the like. The polymers contained in these water-insoluble carriers may be those commercially available, or may be produced by a known method.

The surface of the water-insoluble carrier in the present invention means, when the kurtosis is used as an index, an area of the water-insoluble carrier where irregularity of the surface can be detected in accordance with JIS B 0601:2001 using a laser microscope, or means, when the charge is used as an index, an area of the water-insoluble carrier where a charged functional group(s) is(are) present, or to which a compound(s) containing a charged functional group is(are) bound. For example, in cases where the water-insoluble carrier is porous, the surface layer inside each pore is also included in the surface.

The water-insoluble carrier of the present invention may be in the shape of, for example, a film, a particle, or a fiber. The water-insoluble carrier is especially preferably a fiber or a particle from the viewpoint of easily ensuring blood channels. Furthermore, in cases where the carrier is used for blood purification, it is more preferably a fiber because of its large specific surface area, flexibility that allows deformation, and ease of handling. The water-insoluble carrier is still more preferably a sea-island composite fiber from the viewpoint of the fact that it can be easily processed into a blood purification device while its strength is maintained. Further, the fiber is preferably used after being processed into the form of a nonwoven fabric, a knitted fabric, a woven fabric, or the like from the viewpoint of easily filling the water-insoluble carrier upon its use, and uniformly forming liquid channels.

The water-insoluble carrier which removes activated platelets of the present invention may be used as the water-insoluble carrier alone, or as a product prepared by immobilizing a suitable reinforcing material to, or mixing such a material with, the water-insoluble carrier. The operation of immobilization or mixing may be carried out, for example, before or after processing into the adsorption carrier of a blood purification device or the like.

In cases where the water-insoluble carrier of the present invention is in the form of a fiber or a particle, the carrier preferably has a diameter of not less than 1 $\mu$m. From the viewpoint of ensuring channels though which blood cells can pass, the carrier has a diameter of more preferably not less than 3 $\mu$m, still more preferably not less than 5 $\mu$m. From the viewpoint of ensuring a specific surface area for the adsorption, the carrier has a diameter of preferably not more than 100 $\mu$m, more preferably not more than 60 $\mu$m, still more preferably not more than 40 $\mu$m. Thus, the water-insoluble carrier has a diameter of preferably 1 to 100 $\mu$m, more preferably 3 to 60 $\mu$m, still more preferably 5 to 40 $\mu$m. Any preferable lower limit may be combined with any preferable upper limit.

"Kurtosis" (hereinafter referred to as "Rku") is an index specified by JIS B 0601:2001, and quantifies the sharpness of a height distribution. The term means a parameter whose value allows judgment of the degree of sharpness of the surface shape of a water-insoluble carrier. Rku can be calculated by capturing an image of the surface of the water-insoluble carrier using a laser microscope, and analyzing the image using analysis software. Given the variability of the surface of the water-insoluble carrier, it is appropriate to perform the image analysis for an image extracted from a plane.

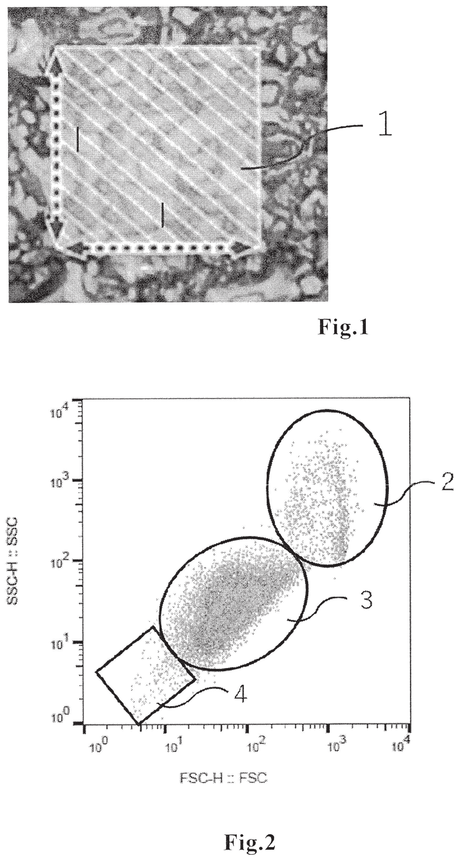

As shown in Fig. 1, from an image of the surface of the water-insoluble carrier obtained using a laser microscope, a part is extracted at an angle with a sampling length l in orthogonal directions. Rku represents the fourth power mean of z(x) in the non-dimensional sampling length obtained by the fourth power of the root-mean-square height (Zq) in this extracted part, and can be calculated according to the following Equation (1) using the surface roughness analysis function (VK Viewer ver.2.5 and VK Analyzer ver.2.5, manufactured by Keyence Corporation) for an image obtained using a laser microscope (for example, an Ultra-Depth Color 3D Shape Measurement Microscope VK-9710, manufactured by Keyence Corporation). Specifically, the measurement is carried out according to "Measurement of Kurtosis" as described later.

$$Rku = \frac{1}{Zq^4} \left[ \frac{1}{N} \sum_{n=1}^{N} Z_n^4 \right] \quad \text{... Equation (1)}$$

The sampling length l for the extraction from the image of the surface of the water-insoluble carrier is 5 to 100 $\mu$m. In cases where the length of the shortest side of the water-insoluble carrier in the captured image is less than 5 $\mu$m or not less than 200 $\mu$m, the sampling length 1 is set to 1/5 to 1/2 of the length of the shortest side. In cases where the water-insoluble carrier is a fiber, the diameter calculated from the area of a cross-section perpendicular to the direction of extension of the fiber may be used as the length of the shortest side. In cases where the water-insoluble carrier is a particle, the diameter of the circle having the same area as the cross-sectional area of a particle in the captured image may be used.

In the removal of activated platelets, when the distribution of the surface height of the water-insoluble carrier is biased toward the upper part (peak) relative to the mean plane, in other words, when the surface has a shape with less irregularities, the activated platelets extend their pseudopodia to promote their aggregation and adhesion to the material, so that the carrier can have improved removal ability. On the other hand, when the height distribution has a sharp, needle-like shape relative to the mean plane, in other words, when the surface has a shape with sharp irregularities, aggregation and adhesion of activated platelets are suppressed, leading to a decrease in the removal ability. Thus, the surface Rku needs to be 0.1 to 16.0, and is preferably 0.1 to 13.0, more preferably 0.1 to 10.0, still more preferably 0.1 to 6.0, especially preferably 0.1 to 3.0. Any preferable lower limit may be combined with any preferable upper limit.

Rku can be controlled by adjusting the temperature at which, or the time for which, the water-insoluble carrier is immersed in a solution in which the water-insoluble carrier can be dissolved, or by adjusting the concentration of a cross-linking agent that suppresses dissolution of the water-insoluble carrier, or by adjusting the temperature at which, or the time for which, the water-insoluble carrier is reacted with the cross-linking agent.

In cases where the water-insoluble carrier is a polymer containing an aromatic ring, such as polystyrene, the cross-linking agent employed may be paraformaldehyde (hereinafter referred to as "PFA") or the like.

The surface of the water-insoluble carrier of the present invention preferably has a charge.

The term "surface has a charge" means that the water-insoluble carrier comprises a positively charged or negatively charged functional group, or comprises a compound containing a positively charged or negatively charged functional group, on its surface. The compound containing a positively charged or negatively charged functional group is not limited as long as the compound is capable of interacting with a substance to be removed such as a growth factor. Regarding the chemical structure of the compound, examples of the compound include compounds containing an amino group, which is a positively charged functional group (cationic functional group), and compounds containing a sulfonic group or a carboxyl group, which is a negatively charged functional group (anionic functional group). The functional group may be used by combining a plurality of the same or different functional groups.

The compound containing a charged functional group may also contain an uncharged functional group as long as the compound contains the charged functional group. For example, a compound in which an alkyl group such as methyl or ethyl; or an aryl group, such as a phenyl group, a phenyl group substituted by an alkyl group (e.g., para(p)-methylphenyl, meta(m)-methylphenyl, ortho(o)-methylphenyl, para(p)-ethylphenyl, meta(m)-ethylphenyl, or ortho(o)-ethylphenyl), or a phenyl group substituted by a halogen atom (e.g., para(p)-fluorophenyl, meta(m)-fluorophenyl, ortho(o)-fluorophenyl, para(p)-chlorophenyl, meta(m)-chlorophenyl, or ortho(o)-chlorophenyl); is bound to a charged functional group is included in the compound containing a charged functional group.

From the viewpoint of obtaining a sufficient interaction with a growth factor, and from the viewpoint of ensuring the freedom of configuration of the introduced functional group to obtain an appropriate interaction with the substance to be removed, the charge amount of the surface of the water-insoluble carrier of the present invention is preferably 0.3 to 3.0 mmol, more preferably 0.5 to 2.0 mmol, still more preferably 0.5 to 1.5 mmol per 1 g dry mass of the water-insoluble carrier. Any preferable lower limit may be combined with any preferable upper limit.

The charge amount of the surface of the water-insoluble carrier can be controlled, for example, by adjusting the type of functional group introduced into the water-insoluble carrier, or adjusting the concentration of the compound containing the charged functional group in the solution or the reaction time upon carrying out the reaction.

From the viewpoint of excellent biocompatibility, the water-insoluble carrier of the present invention preferably contains an amino group on the surface. Examples of the compound containing an amino group include compounds containing an amino group derived from a primary amine such as methylamine, ethylamine, propylamine, butylamine, pentylamine, hexylamine, heptylamine, octylamine, or dodecylamine; compounds containing an amino group derived from a secondary amine such as methylhexylamine, diphenylmethylamine, or dimethylamine; compounds containing an amino group derived from an amine that contains an unsaturated alkyl chain such as allylamine, or containing an amino group derived from a tertiary amine such as trimethylamine, triethylamine, dimethylethylamine, phenyldimethylamine, or dimethylhexylamine; compounds containing an amino group derived from an amine that contains an aromatic ring such as 1-(3-aminopropyl)imidazole, pyridin-2-amine, or 3-sulfoaniline; and compounds containing two or more amino groups bound to an alkyl chain, an aromatic compound, a heterocyclic compound, a homocyclic compound, or the like (hereinafter referred to as "polyamine"), such as tris(2-aminoethyl)amine, ethylenediamine, diethylenetriamine, triethylenetetramine, tetraethylenepentamine (hereinafter referred to as "TEPA"), dipropylenetriamine, polyethyleneimine, N-methyl-2,2'-diaminodiethylamine, N-acetyl-ethylenediamine, and 1,2-bis(2-aminoethoxyethane). From the viewpoint of efficiently adsorbing growth factors released from activated platelets, the amino group contained on the surface of the water-insoluble carrier is preferably an amino group derived from a polyamine. Further, from the viewpoint of increasing the freedom of configuration of the functional group, and increasing the efficiency of adsorption of growth factors released from activated platelets, the amino group is more preferably an amino group derived from a primary amine or secondary amine.

The compound containing a sulfonic group is not limited as long as it contains at least one sulfonic group, and examples of the compound include aliphatic sulfonic acids such as sulfonic acid and methanesulfonic acid; aromatic sulfonic acids such as benzenesulfonic acid, para(p)-phenol sulfonic acid, and 4-methylbenzenesulfonic acid; and halosulfonic acids such as fluorosulfonic acid and chlorosulfonic acid.

The compound containing a carboxyl group is not limited as long as it contains at least one carboxyl group, and examples of the compound include aliphatic carboxyl groups such as acetic acid and propionic acid; and aromatic carboxyl groups such as benzenecarboxyl groups.

The water-insoluble carrier and the compound containing the charged functional group may be bound directly, or may be bound through a spacer derived from a reactive functional group between the water-insoluble carrier and the compound containing the charged functional group. The spacer is not limited as long as it has an electrically neutral chemical bond such as an amide bond, a urea bond, an ether bond, or an ester bond. The spacer preferably has an amide bond or a urea bond.

Examples of the reactive functional group mediating the bonding between the water-insoluble carrier and the compound containing the charged functional group include activated halogen groups, such as halomethyl groups, haloacetyl groups, haloacetamidomethyl groups, and alkyl halide groups; epoxide groups, carboxyl groups, isocyanate groups, thioisocyanate groups, and acid anhydride groups. In particular, activated halogen groups are preferred, and haloacetamidomethyl groups are more preferred, from the viewpoint of the fact that they have appropriate reactivity.

The reactive functional group can be bound to the water-insoluble carrier by reacting the water-insoluble carrier with an appropriate reagent in advance. For example, in cases where the water-insoluble carrier is polystyrene, and the reactive functional group is a chloroacetamidomethyl group, a polystyrene to which the chloroacetamidomethyl group is bound can be obtained by reacting the polystyrene with N-hydroxymethyl-2-chloroacetamide (hereinafter referred to as "NMCA"). By reacting the polystyrene to which the chloroacetamidomethyl group is bound, with, for example, TEPA, which contains an amino group, a polystyrene to which the TEPA is bound through the acetamidomethyl group can be obtained. In this case, TEPA is the compound containing the charged functional group. The starting material and the reagents used for the production of the water-insoluble carrier which removes activated platelets may be those commercially available or produced by known methods.

Since the compound containing the charged functional group is required to interact with the substances to be removed in blood, the compound is preferably bound to at least a surface which comes into contact with blood, among the surfaces of the water-insoluble carrier.

The charge amount of the water-insoluble carrier can be measured by an acid-base titration method using an aqueous hydrochloric acid or sodium hydroxide solution. Specifically, the measurement can be carried out by the method described below in "Measurement of Positive Charge Amount" or "Measurement of Negative Charge Amount". The charge amount, either the positive charge amount or the negative charge amount, is expressed in an absolute value.

The "fiber diameter" can be determined by the following method. First, 100 dried fiber samples are randomly collected, and, for each sample, one photograph of a cross-section perpendicular to the direction of extension of the fiber is taken using a scanning electron microscope or the like at a magnification of 1000 to 3000. The diameter of each fiber cross-section is then measured, and the average of the measured values (the average of the diameters of cross-sections of a total of 100 fibers) is calculated. By this, the "fiber diameter" can be determined. In cases where the fiber cross-section is not circular, the diameter of the circle having the same area as the cross-sectional area is regarded as the fiber diameter.

The "particle diameter" can be determined by the following method. First, 10 sample groups of dried particles are randomly collected, and, for each sample, one photograph is taken using a scanning electron microscope or the like at a magnification of 1000 to 3000. Subsequently, for each photograph, the diameters of 10 particles are measured, and the average of the measured values (the average of the diameters of a total of 100 particles) is calculated. By this, the "particle diameter" can be determined. In cases where the shape of a photographed particle is not circular, the diameter of the circle having the same area as the particle area in the photograph is regarded as the particle diameter.

The "blood" means a liquid containing a protein, a lipid, a blood cell component, or the like. Specific examples of the blood include a liquid prepared by adding a protein, a lipid, a blood cell component, and/or the like to a buffer solution; a body fluid; blood; plasma; and serum.

The "blood component" means a component constituting blood. Examples of the blood component include blood cell components such as erythrocytes, leukocytes, and platelets; and humoral factors such as growth factors released from cells. In cases where treatment is carried out for the purpose of suppressing thrombus formation, it is preferred to remove growth factors, among the humoral factors, in addition to activated platelets.

The "activated platelets" means platelets having a function that causes thrombus formation in response to bleeding, inflammation, immunity, phylaxis, arteriosclerosis, cancer metastasis, or the like, which thrombus formation occurs by aggregation of those platelets due to extension of their amoeboid processes called pseudopodia.

The degree of activation of the platelets can be determined by measuring the expression of surface antigens of those platelets by flow cytometry or the like. From the viewpoint of quantitative evaluation of the platelet activation, a method based on quantitative measurement of the expression of surface antigens of the platelets by flow cytometry or the like is superior to a method based on subjective morphological judgment of the pseudopodial extension, deformation, adhesion, aggregation, or the like of the platelets.

As surface antigens of platelets, CD41, CD42, CD42b, CD61, CD62P, and the like are known. From the viewpoint of detecting activated platelets, a method based on detection of CD62P is preferred since CD62P is specifically expressed on the platelet membrane upon the platelet activation. In some cases, activated platelets are bound to erythrocytes or leukocytes. However, the "activated platelets" in the present description means activated platelets not bound to erythrocytes or leukocytes.

The "non-activated platelets" means platelets for which CD62P is not detected by flow cytometry or the like, or platelets having no thrombogenic function since the extension of their amoeboid processes called pseudopodia does not occur, and hence the deformation, adhesion, or aggregation of those platelets does not occur. The degree of inactivation can be determined by measuring the expression of surface antigens of the platelets by flow cytometry or the like.

The concentration of the activated platelets can be calculated by, for example, reacting a platelet fraction derived from peripheral blood with an activation detection reagent that specifically binds to activated platelets (activated platelet binding reagent), and measuring the fraction that has specifically bound to the activated platelet binding reagent in the platelet fraction derived from peripheral blood.

The activated platelet detection reagent does not bind to non-activated platelets, but has the binding ability with activated platelets. For example, activated platelets can be detected by using an anti-CD62P antibody (for example, Anti-human CD62P (P-Selectin) Antibody, BioLegend) that detects CD62P, which is known as a cell surface marker specific to activated platelets.

The water-insoluble carrier of the present invention is characterized in that it removes activated platelets. The removal of activated platelets means that the value obtained by dividing the activated platelet removal rate of the water-insoluble carrier [A] by the non-activated platelet removal rate of the water-insoluble carrier [B], that is, the activated platelet removal ratio, is higher than 1.5 ([A] / [B] > 1.5). The higher the activated platelet removal ratio, the more selectively the activated platelets are removed. Thus, the activated platelet removal ratio is preferably not less than 2.0, more preferably not less than 3.0, still more preferably not less than 5.0.

From the viewpoint of suppressing thrombus formation, the activated platelet removal rate is preferably not less than 20%, more preferably not less than 30%, still more preferably not less than 40%, especially preferably not less than 60%.

The "growth factor" means a molecule having a physiological activity that promotes the growth of cells or a tissue. Examples of a growth factor useful in the present invention include platelet-derived growth factor (hereinafter referred to as PDGF), vascular endothelial growth factor (hereinafter referred to as "VEGF"), fibroblast growth factor (hereinafter referred to as FGF), and mixtures of these factors. PDGF includes PDGF-AA, PDGF-BB, PDGF-AB, PDGF-CC, and PDGF-DD. VEGF includes VEGF-A, VEGF-B, VEGF-C, VEGF-D, and VEGF-E. FGF includes FGF1 to 23. The above growth factors may be bound to other proteins.

The water-insoluble carrier of the present invention preferably removes PDGF, VEGF, and/or FGF. In cases where PDGF, VEGF, and/or FGF is/are removed together with activated platelets, thrombus formation can be suppressed, and in addition, the progression of pathological conditions in which PDGF, VEGF, and/or FGF is/are involved can be suppressed. Examples of the pathological conditions in which PDGF, VEGF, and/or FGF is/are involved include arteriosclerosis, cancers, inflammatory diseases, infections, and kidney diseases. From the viewpoint of suppressing the progression of the pathological conditions, the removal rate(s) of PDGF, VEGF, and/or FGF is/are preferably not less than 20%, more preferably not less than 50%, still more preferably not less than 70%, especially preferably not less than 80%.

The "blood purification device" means a device that removes substances from blood, to purify the blood.

The blood purification device of the present invention is characterized in that it comprises the water-insoluble carrier of the present invention. The shape of the device is not limited as long as blood purification can be carried out therewith. Examples of a blood purification device that can be suitably used include a blood purification column, a blood purification filter, a blood purification bag, a blood purification unit, a blood purification case, and a blood purification syringe.

The "blood purification column" means a blood purification device at least comprising a blood inlet, a blood outlet, and a case section, wherein the case comprises a water-insoluble carrier therein.

The blood purification column of the present invention is characterized in that it comprises the water-insoluble carrier of the present invention. Regarding the shape of the blood purification column, for example, a radial-flow-type column may be suitably used.

The "thrombus" means a clot generated by aggregation of platelets in blood, or clotting of blood. Narrowing of the inside of a blood vessel caused by a thrombus leads to decreased circulation of blood, resulting in circulatory disturbance and organ failure. Furthermore, in cases where the blood flow stops, the organs located downstream cannot receive nutrients, resulting in necrosis of cells to cause functional impairment.

The blood purification device comprising the water-insoluble carrier of the present invention can be suitably used for suppression of thrombus formation. The use of the blood purification device is not limited as long as it is used for the suppression of thrombus formation. For example, the device may be used for diseases caused by thrombi, including both venous thrombosis, in which a thrombus is formed in a vein, and arterial thrombosis, in which a thrombus is formed in an artery.

Examples of diseases for which suppression of thrombus formation can be expected to be therapeutically effective include atheromatous disease/arteriosclerosis, myocardial infarction, ischemic heart disease, cerebral infarction, stroke, valvular heart disease, pulmonary embolism, angina pectoris, dementia, hyperlipidemia, dyslipidemia, diabetes, economy-class syndrome, artery obstruction, deep vein thrombosis, portal vein thrombosis, intermittent claudication, limb numbness, deafness, pain, sepsis, disseminated intravascular coagulation (DIC), bacteremia, viral infection, toxin infection, COVID-19 infection, acute lung injury (ALI), acute respiratory distress syndrome (ARDS), pneumonia, acute respiratory failure, septic shock, toxic shock syndrome, multiple organ failure, chronic obstructive pulmonary disease, Kawasaki disease, and cancers.

EXAMPLES

The present invention will now be specifically described with reference to Experimental Examples and Comparative Examples, but the present invention is not limited to these examples. First, a measurement method and an evaluation method are described below. Although the following is one example showing a case where the water-insoluble carrier is a knitted fabric, the same method may be used for the measurement also in cases where the water-insoluble carrier is not in the form of a knitted fabric

(Measurement of Kurtosis)

About 0.3 g of a water-insoluble carrier was taken out, and dried under vacuum at 15 to 40°C for not less than 8 hours. The vacuum-dried water-insoluble carrier was fixed on the observation stage of a laser microscope (Ultra-Depth Color 3D Shape Measurement Microscope VK-9710, manufactured by Keyence Corporation) using a double-stick tape. A long-distance lens (type: EPIPLAN/ELWD100; manufactured by Keyence Corporation) was used as an objective lens to provide a magnification of ×100. The optical zoom was set to ×1, and no neutral density filter was used. Images of the surface of the water-insoluble carrier were taken using the laser microscope, and the surface roughness analysis function (VK Viewer ver.2.5 and VK Analyzer ver.2.5, manufactured by Keyence Corporation) was used to perform calculation of Rku according to the following Equation (1).

Here, Zq means the root-mean-square height, and is represented as the following Equation (2). In the analysis of the image taken with the laser microscope for the calculation of Rku, the obtained value may vary depending on the conditions. Therefore, the analysis was carried out as follows.

For the analysis, the method described in JIS B 0601:2001 was used employing the surface roughness, and without carrying out waviness correction. Images were captured from three sites, and 10 fields of view were analyzed for the image captured from each site. Thus, images from a total of 30 fields of view were analyzed. For each field of view, an area extracted with a sampling length 1 of 10 $\mu$m (length) $\times$ 10 $\mu$m (width) was analyzed.

Since the surface may show variation, the Rku value of each sample of the water-insoluble carrier was determined as the average for the 30 fields of view. However, since occurrence of variation in the surface shape of the water-insoluble carrier may disturb appropriate evaluation, the average was calculated after excluding outliers.

For judgment of the outliers, the interquartile range (IQR) was used. The quartile points were calculated from the Rku values of the 30 fields of view. Each outlier was defined as "a value that is not less than the value obtained by adding 1.5 times the interquartile range to the third quartile" or "a value that is not more than the value obtained by subtracting 1.5 times the interquartile range from the first quartile", and excluded from the calculation of the average. In cases where there were not less than seven fields of view showing outliers, the operation was carried out again from the capturing of the images using the laser microscope, and then the same analysis was carried out. The Rku value was rounded to one decimal place.

$$Rku = \frac{1}{Zq^4} \left[ \frac{1}{N} \sum_{n=1}^{N} Z_n^4 \right] \quad \text{... Equation (1)}$$

$$Zq = \sqrt{\frac{1}{N} \sum_{n=1}^{N} Z_n^2} \quad \text{... Equation (2)}$$

(Measurement of Positive Charge Amount)

The positive charge amount of the water-insoluble carrier was calculated by performing acid-base back titration of cationic functional groups in the water-insoluble carrier.

A knitted fabric as a water-insoluble carrier was punched out using a puncher (diameter: 28 mm) to obtain six pieces. The punched-out water-insoluble carrier was placed in a polypropylene centrifuge tube (50 mL). Subsequently, 6 mol/L aqueous sodium hydroxide solution was added thereto to immerse the water-insoluble carrier therein, and then the resulting mixture was stirred by inversion using a rotator for not less than 30 minutes (desalting treatment for the positive charge). The 6 mol/L aqueous sodium hydroxide solution was discarded from the centrifuge tube, and then ion-exchanged water was added to the tube to immerse the water-insoluble carrier, followed by stirring the resulting mixture by vortexing for not less than 1 second, and then discarding the washing liquid. This washing operation was repeated not less than five times, and then the neutrality of the pH of the last washing liquid was confirmed using phenolphthalein solution.

Subsequently, the water-insoluble carrier was placed in a vacuum dryer, and dried at 15 to 40°C. The end point of the vacuum drying was set to not less than 8 hours after the start of drying. The vacuum drying was terminated when the mass reduction rate as determined by weighing using an electronic balance was less than 5% between the first measurement, and the second measurement that was carried out not less than 15 minutes after the first measurement. In cases where the mass reduction rate was found to be not less than 5%, additional vacuum drying was carried out for not less than 15 minutes. The same operation was repeated until the mass reduction rate became less than 5%, and thereafter, the dry mass [a] of the water-insoluble carrier was determined.

The vacuum-dried water-insoluble carrier was then placed in a polypropylene centrifuge tube (50 mL), and 25 mL of 0.1 mol/L hydrochloric acid [b] was added thereto, followed by stirring the resulting mixture by inversion using a rotator for 30 minutes. The solution [c] after the stirring by inversion was aliquoted in 5-mL volumes into centrifuge tubes, and then 30 μL each of methyl red solution and phenolphthalein solution were added thereto to provide a measurement solution. The measurement solution was prepared in three tubes.

Subsequently, titration was carried out with 0.05 mol/L aqueous sodium hydroxide solution [d], and the endpoint was set to the time point when the measurement solution turned yellow after three times of mixing by inversion. The titration measurement was carried out for the three tubes of the measurement solution. The average of the drop volume of the aqueous sodium hydroxide solution used for the titration was defined as the average drop volume [e], and the positive charge amount was calculated using the following Equation (3).

Positive charge amount per 1 g dry mass (mmol/g) = (aliquot volume of [c] × concentration of [b] / concentration of [d] - [e]) × concentration of [d] × (amount of [b] added / aliquot volume of [c]) / [a]     Equation (3)

The positive charge amount was rounded to one decimal place.

(Measurement of Negative Charge Amount)

The negative charge amount of the water-insoluble carrier was calculated by performing acid-base back titration of anionic functional groups in the water-insoluble carrier.

A knitted fabric as a water-insoluble carrier was punched out using a puncher (diameter: 28 mm) to obtain six pieces. The punched water-insoluble carrier was placed in a polypropylene centrifuge tube (50 mL). Subsequently, 6 mol/L hydrochloric acid was added thereto to immerse the water-insoluble carrier therein, and then the resulting mixture was stirred by inversion using a rotator for not less than 30 minutes (desalting treatment for the negative charge). The 6 mol/L hydrochloric acid was discarded from the centrifuge tube, and then ion-exchanged water was added to the tube to immerse the water-insoluble carrier, followed by stirring the resulting mixture by vortexing for not less than 1 second, and then discarding the washing liquid. This washing operation was repeated not less than five times, and then the neutrality of the pH of the last washing liquid was confirmed using methyl red solution.

Subsequently, the water-insoluble carrier was placed in a vacuum dryer, and dried at 15 to 40°C. The end point of the vacuum drying was set to not less than 8 hours after the start of drying. The vacuum drying was terminated when the mass reduction rate as determined by weighing using an electronic balance was less than 5% between the first measurement, and the second measurement that was carried out not less than 15 minutes after the first measurement. In cases where the mass reduction rate was found to be not less than 5%, additional vacuum drying was carried out for not less than 15 minutes. The same operation was repeated until the mass reduction rate became less than 5%, and thereafter, the dry mass [f] of the water-insoluble carrier was determined.

The vacuum-dried water-insoluble carrier was then placed in a polypropylene centrifuge tube (50 mL), and 25 mL of 0.1 mol/L aqueous sodium hydroxide solution [g] was added thereto, followed by stirring the resulting mixture by inversion using a rotator for 30 minutes. The solution [h] after the stirring by inversion was aliquoted in 5-mL volumes into centrifuge tubes, and then 5 mL of 0.1 mol/L hydrochloric acid [i] was further added thereto, followed by adding 30 μL each of methyl red solution and phenolphthalein solution thereto to provide a measurement solution. The measurement solution was prepared in three tubes.

Subsequently, titration was carried out with 0.05 mol/L aqueous sodium hydroxide solution [j], and the endpoint was set to the time point when the measurement solution turned yellow after three times of mixing by inversion. The titration measurement was carried out for the three tubes of the measurement solution. The average of the drop volume of the aqueous sodium hydroxide solution used for the titration was defined as the average drop volume [k], and the negative charge amount was determined using the following Equation (4).

| Negative charge amount per 1 g dry mass (mmol/g) = [k] $\times$ concentration of [j] $\times$ (amount of [g] added / aliquot volume of [h]) / [f] | Equation (4) |
|---|---|

The negative charge amount was rounded to one decimal place.

(Measurement of Fiber Diameter)

First, from a knitted fabric that is a dried water-insoluble carrier, 100 fibers were randomly collected. For each sample, one photograph of a cross-section perpendicular to the direction of extension of the fiber was taken using a scanning electron microscope at a magnification of 2000. Subsequently, for each fiber cross-section, the cross-sectional area was determined using analysis software (Photoshop Elements.14.0, manufactured by Adobe), and the diameter of the circle having the same area as the obtained cross-sectional area was calculated. The average of the resulting values (the average of the diameters of cross-sections of a total of 100 fibers) was then determined as the fiber diameter. The fiber diameter was rounded to the nearest integer.

(Measurement of Removal Rates of Activated Platelets and Non-Activated Platelets)

The water-insoluble carrier was cut into disks with a diameter of 1 cm, and the disks were stacked and packed into a cylindrical column (1 cm (inner diameter) $\times$ 1.2 cm (height); internal volume, 0.94 cm$^3$; outer diameter, 2 cm; made of polycarbonate) having a solution inlet and a solution outlet, to prepare a column.

Heparin was added to blood of a healthy human volunteer to 5 units/mL. Subsequently, adenosine diphosphate (ADP) was added to the blood of the healthy human volunteer to 2 $\mu$mol/L, and then the blood was shaken in a hot water bath under conditions at 37°C at 65 rpm for 30 minutes to activate the blood.

The activated blood was then passed through the column at a flow rate of 0.63 mL/minute using a pump. The blood was collected at the column inlet and outlet. Regarding the sample at the column inlet, the blood that had been immersed in the hot water bath was collected before being passed through the column. Regarding the sample at the column outlet, the time point at which the blood flowed into the column after the beginning of passing the blood was regarded as Minute 0, and the blood that had flowed out from the column outlet between Minute 3.5 and Minute 6.5 was collected to provide the sample.

The obtained samples were stained for surface antigens of platelets using the fluorescently labeled antibodies listed in Table 1. Further, the cells were fixed by addition of 0.1% by mass PFA solution, and then cooled on ice, followed by storage in a dark place. The number of cells contained in each sample was then immediately counted. The number of platelets was counted using an automated hematology analyzer XT-1800i (manufactured by Sysmex Corporation). For the measurement of the surface antigens, a flow cytometer (BD Cytometer Setup And Tracking Beads, manufactured by Becton, Dickinson and Company) was used.

For the analysis, BD FACS Diva (registered trademark) software Version 6.1.3 (manufactured by Becton, Dickinson and Company) or FLOWJO (manufactured by Tomy Digital Biology Co., Ltd.) was used. The ratio of activated platelets was calculated according to the following Equation (5); the ratio of non-activated platelets was calculated according to the following Equation (6); the concentration of activated platelets was calculated according to the following Equation (7); and the concentration of non-activated platelets was calculated according to the following Equation (8). Using the obtained values, the activated platelet removal rate, the non-activated platelet removal rate, and the activated platelet removal ratio were calculated according to the following Equations (9), (10), and (11), respectively.

| Ratio of activated platelets (%) = number of CD62-positive cells in platelet fraction of CD41-positive cells / number of platelets among CD41-positive cells $\times$ 100 | Equation (5) |
|---|---|

| Ratio of non-activated platelets (%) = number of CD62-negative cells in platelet fraction of CD41-positive cells / number of platelets among CD41-positive cells $\times$ 100 | Equation (6) |
|---|---|

| Concentration of activated platelets (cells/$\mu$L) = number of platelets $\times$ ratio of activated platelets / 100 | Equation (7) |
|---|---|

| Concentration of non-activated platelets (cells/$\mu$L) = number of platelets $\times$ ratio of non-activated platelets / 100 | Equation (8) |
|---|---|

Activated platelet removal rate (%) = {(concentration of activated platelets on column inlet side) - (concentration of activated platelets on column outlet side)} / (concentration of activated platelets on column inlet side) × 100    Equation (9)

Non-activated platelet removal rate (%) = {(concentration of non-activated platelets on column inlet side) - (concentration of non-activated platelets on column outlet side)} / (concentration of non-activated platelets on column inlet side) × 100    Equation (10)

Activated platelet removal ratio = activated platelet removal rate / non- activated platelet removal rate    Equation (11)

The activated platelet removal rate and the non-activated platelet removal rate were rounded to the nearest integers. The activated platelet removal ratio was rounded to one decimal place.

Here, the platelet fraction of CD41-positive cells means the Fraction 3 shown in Fig. 2 as identified by analyzing a CD41-positive cell population by plotting the forward-scattered light (FSC) on the abscissa and plotting the side-scattered light (SSC) on the ordinate, and excluding, on the cell-population-basis, the fraction of microparticles of finely fragmented platelets (Fraction 4) and the fraction of platelets bound to erythrocytes (Fraction 2).

[Table 1]

| Measurement target | Antibody name | Manufacturer | Catalog number |
|---|---|---|---|
| Platelets | PE Mouse IgG1, κ Isotype Ctrl (FC) Antibody | BioLegend | 400113 |
| Platelets | PE anti-human CD41 Antibody | BioLegend | 303706 |
| CD62P-positive cells | APC Mouse IgG1, κ Isotype Ctrl Antibody | BioLegend | 400120 |
| CD62P-positive cells | APC anti-human CD62P (P-Selectin) Antibody | BioLegend | 304910 |

(Measurement of PDGF Removal Rate)

The thickness of the water-insoluble carrier was measured using a micrometer (CLM2-10QMB or CLM2-10QMX, manufactured by Mitutoyo Corporation), and the volume of the knitted fabric was calculated according to the following Equation (12).

Subsequently, four punched-out pieces of the knitted fabric with a diameter of 8 mm were placed in each polypropylene container. Fetal bovine serum (hereinafter referred to as "FBS") that had been supplemented with PDGF (product number: 220-BB; manufactured by R&D Systems, Inc.) to a concentration of 1000 pg/mL and inactivated (inactivation conditions: 56°C, 2 hours of immersion) was added to this container such that the volume of the knitted fabric per liquid volume was 0.12 $cm^3$/mL, and the resulting mixture was mixed by inversion using a rotator under rotation conditions at 27 rpm in an incubator at 37°C for 24 hours. In addition, a Blank was prepared by mixing FBS by inversion in an incubator at 37°C for 24 hours in the same manner except that the knitted fabric was not placed in the container.

Using an ELISA kit (product number: DBB00; manufactured by R&D Systems, Inc.), the PDGF concentrations in the Blank, and in the FBS after mixing by inversion were measured, and then the PDGF removal rate was calculated according to the following Equation (13).

Volume of knitted fabric ($cm^3$) = (diameter of punched-out knitted fabric / 2)$^2$ × $\pi$ × thickness of knitted fabric × number of pieces    Equation (12)

PDGF Removal Rate (%) = {(PDGF concentration in Blank) - (PDGF concentration after mixing by inversion)} / (PDGF concentration in Blank) × 100    Equation (13)

The volume of the knitted fabric was rounded to two decimal places, and the PDGF removal rate was rounded to the nearest integer.

(Measurement of VEGF Removal Rate)

The thickness of the water-insoluble carrier was measured using a micrometer (CLM2-10QMB or CLM2-10QMX, manufactured by Mitutoyo Corporation), and the volume of the knitted fabric was calculated according to Equation (12).

Subsequently, four punched-out pieces of the knitted fabric with a diameter of 8 mm were placed in each polypropylene container. Fetal bovine serum (hereinafter referred to as "FBS") that had been supplemented with VEGF (product number: 293-VE/CF; manufactured by R&D Systems, Inc.) to a concentration of 1000 pg/mL and inactivated (inactivation conditions: 56°C, 2 hours of immersion) was added to this container such that the volume of the knitted fabric per liquid volume was 0.12 cm$^3$/mL, and the resulting mixture was mixed by inversion using a rotator under rotation conditions at 27 rpm in an incubator at 37°C for 24 hours. In addition, a Blank was prepared by mixing FBS by inversion in an incubator at 37°C for 24 hours in the same manner except that the knitted fabric was not placed in the container.

Using an ELISA kit (product number: DVE00; manufactured by R&D Systems, Inc.), the VEGF concentrations in the Blank, and in the FBS after mixing by inversion were measured, and then the VEGF removal rate was calculated according to the following Equation (14).

VEGF Removal Rate (%) = {(VEGF concentration in Blank) - (VEGF concentration after mixing by inversion)} / (VEGF concentration in Blank) $\times$ 100    Equation (14)

The volume of the knitted fabric was rounded to two decimal places, and the VEGF removal rate was rounded to the nearest integer.

(Measurement of FGF Removal Rate)

The thickness of the water-insoluble carrier was measured using a micrometer (CLM2-10QMB or CLM2-10QMX, manufactured by Mitutoyo Corporation), and the volume of the knitted fabric was calculated according to the following Equation (12). Subsequently, four punched-out pieces of the knitted fabric with a diameter of 8 mm were placed in each polypropylene container. Normal human serum (product number: 12181201; Cosmo Bio Co., Ltd.) supplemented with FGF (product number: 230-00791, manufactured by Ray Biotech, Inc.) to a concentration of 10,000 pg/mL was added to this container such that the volume of the knitted fabric per liquid volume was 0.12 cm$^3$/mL, and the resulting mixture was mixed by inversion using a rotator under rotation conditions at 27 rpm in an incubator at 37°C for 1 hour. In addition, a Blank was prepared by mixing normal human serum by inversion in an incubator at 37°C for 1 hour in the same manner except that the knitted fabric was not placed in the container.

Using an ELISA kit (product number: ELH-bFGF, manufactured by Ray Biotech, Inc.), the FGF concentrations in the Blank, and in the normal human serum after mixing by inversion were measured, and then the FGF removal rate was calculated according to the following Equation (15).

FGF Removal Rate (%) = {(FGF concentration in Blank) - (FGF concentration after mixing by inversion)} / (FGF concentration in Blank) $\times$ 100    Equation (15)

The volume of the knitted fabric was rounded to two decimal places, and the FGF removal rate was rounded to the nearest integer.

(Preparation of Knitted Fabric)

Using the following components under yarn-making conditions at a spinning rate of 1250 m/minute, 36 filaments of a sea-island composite fiber (fiber diameter: 3 dtex, 20 $\mu$m) with 704 islands per filament were bundled to obtain a fiber.

Island component: polypropylene
Sea component: polystyrene
Composite ratio (mass ratio): island:sea = 50:50
Using a tube knitting machine (machine name: Circular Knitting Machine MR-1, Maruzen Sangyo Co., Ltd.), the obtained fiber was weft-knitted to prepare a knitted fabric with an areal weight of 70 g/m$^2$.

(Example 1)

(Preparation of Chloroacetamidomethylated Knitted Fabric)

After adding 3.3 g of NMCA to a mixture of 26 mL of nitrobenzene and 17 mL of 98% by mass sulfuric acid, the resulting mixture was stirred at 10°C to dissolve the NMCA, to prepare an NMCA solution. Subsequently, 0.2 g of PFA was added to a mixture of 2 mL of nitrobenzene and 1.3 mL of 98% by mass sulfuric acid, and the resulting mixture was stirred at 20°C to dissolve the PFA, to prepare a PFA solution. After cooling 3.3 mL of the PFA solution to 5°C, the PFA solution was mixed with 43 mL of the NMCA solution, to prepare Mixture A. The mixture was stirred for 5 minutes, and then 1 g of the knitted fabric was added thereto, followed by allowing impregnation for 0.25 hours. The impregnated knitted fabric was immersed in 43 mL of nitrobenzene at 10°C to stop the reaction, and then the nitrobenzene adhering to the knitted fabric was washed away with methanol, followed by immersing the knitted fabric in water for washing, to obtain Knitted Fabric A.

(Example 2)

The same operation as in Example 1 was carried out except that the time of impregnation of the knitted fabric in Mixture A in the preparation of the chloroacetamidomethylated knitted fabric was 0.5 hours, to obtain Knitted Fabric B.

(Example 3)

The same operation as in Example 1 was carried out except that the time of impregnation of the knitted fabric in Mixture A in the preparation of the chloroacetamidomethylated knitted fabric was 1 hour, to obtain Knitted Fabric C.

(Example 4)

The same operation as in Example 1 was carried out except that the time of impregnation of the knitted fabric in Mixture A in the preparation of the chloroacetamidomethylated knitted fabric was 1.5 hours, to obtain Knitted Fabric D.

(Example 5)

The same operation as in Example 1 was carried out except that the time of impregnation of the knitted fabric in Mixture A in the preparation of the chloroacetamidomethylated knitted fabric was 2 hours, to obtain Knitted Fabric E.

(Comparative Example 1)

The same operation as in Example 1 was carried out except that the time of impregnation of the knitted fabric in Mixture A in the preparation of the chloroacetamidomethylated knitted fabric was 4 hours, to obtain Knitted Fabric F.

(Example 6)

(Production of Tetraethylenepentamine-Parachlorophenylated Knitted Fabric)

The chloroacetamidomethylated Knitted Fabric A after the washing with methanol, obtained in Example 1 was added as it is to a mixture prepared by dissolving 1.5 mL of TEPA and 2.9 mL of triethylamine in 40 mL of dimethyl sulfoxide (hereinafter referred to as "DMSO"), followed by allowing impregnation at 40°C for 3 hours. The knitted fabric was subjected to filtration through a glass filter, and then washed with 40 mL of DMSO. Subsequently, under a nitrogen atmosphere, 0.1 g of parachlorophenyl isocyanate was added to 25 mL of DMSO that had been dehydrated and dried with activated molecular sieves 3A, and the resulting mixture was warmed to 30°C, followed by adding the whole amount of the washed chloroacetamidomethylated knitted fabric, and allowing impregnation for 1 hour. The knitted fabric was then separated by filtration using a glass filter, and immersed in DMSO in the same amount as the reaction liquid, followed by immersion in methanol and washing, and then immersion in water and washing, to obtain Knitted Fabric G.

(Example 7)

The same operation as in Example 6 was carried out except that the knitted fabric in the preparation of the tetraethylenepentamine-parachlorophenylated knitted fabric was Knitted Fabric B after the washing with methanol, to obtain Knitted Fabric H.

(Example 8)

The same operation as in Example 6 was carried out except that the knitted fabric in the preparation of the tetraethylenepentamine-parachlorophenylated knitted fabric was Knitted Fabric C after the washing with methanol, to obtain Knitted Fabric I.

(Example 9)

The same operation as in Example 6 was carried out except that the knitted fabric in the preparation of the tetraethylenepentamine-parachlorophenylated knitted fabric was Knitted Fabric D after the washing with methanol, to obtain Knitted Fabric J.

(Example 10)

The same operation as in Example 6 was carried out except that the knitted fabric in the preparation of the tetraethylenepentamine-parachlorophenylated knitted fabric was Knitted Fabric E after the washing with methanol, to obtain Knitted Fabric K.

(Example 11)

The same operation as in Example 7 was carried out except that the amount of TEPA added in the preparation of the tetraethylenepentamine-parachlorophenylated knitted fabric was 0.5 mL, to obtain Knitted Fabric L.

(Example 12)

The same operation as in Example 7 was carried out except that the amount of TEPA added in the preparation of the tetraethylenepentamine-parachlorophenylated knitted fabric was 2 mL, to obtain Knitted Fabric M.

(Example 13)

The same operation as in Example 7 was carried out except that the amount of TEPA added in the preparation of the tetraethylenepentamine-parachlorophenylated knitted fabric was 4 mL, to obtain Knitted Fabric N.

(Example 14)

The same operation as in Example 7 was carried out except that the amount of TEPA added in the preparation of the tetraethylenepentamine-parachlorophenylated knitted fabric was 6 mL, to obtain Knitted Fabric O.

(Example 15)

(Preparation of Sulfonated Chloroacetamidomethylated Knitted Fabric)

After adding 3.3 g of NMCA to a mixture of 26 mL of nitrobenzene and 17 mL of 98% by mass sulfuric acid, the resulting mixture was stirred at 10°C to dissolve the NMCA, to prepare an NMCA solution. Subsequently, 0.2 g of PFA was added to a mixture of 2 mL of nitrobenzene and 1.3 mL of 98% by mass sulfuric acid, and the resulting mixture was stirred at 20°C to dissolve the PFA, to prepare a PFA solution. Mixture A was prepared by mixing 3.3 mL of the PFA solution with 43 mL of the NMCA solution, and then warmed to 40°C. The mixture was stirred for 5 minutes, and then 1 g of the knitted fabric was added thereto, followed by allowing impregnation for 0.25 hours to perform sulfonation reaction. The impregnated knitted fabric was immersed in 43 mL of nitrobenzene at 10°C to stop the reaction, and then the nitrobenzene adhering to the knitted fabric was washed away with methanol, followed by immersing the knitted fabric in water for washing, to obtain Knitted Fabric P.

(Example 16)

The same operation as in Example 7 was carried out except that the amount of TEPA added in the preparation of the tetraethylenepentamine-parachlorophenylated knitted fabric was 10 mL, to obtain Knitted Fabric Q.

14

(Example 17)

The same operation as in Example 7 was carried out except that the amount of TEPA added in the preparation of the tetraethylenepentamine-parachlorophenylated knitted fabric was 30 mL, to obtain Knitted Fabric R.

Knitted Fabrics A to R obtained as described above were subjected to measurement of the Rku, the fiber diameter, the activated platelet removal rate, the non-activated platelet removal rate, the PDGF removal rate, the VEGF removal rate, and he FGF removal rate by the above-described methods. In addition, Knitted Fabrics A to O and Knitted Fabrics Q to R were subjected to measurement of the positive charge amount, and Knitted Fabric P was subjected to measurement of the negative charge amount, to determine the absolute values of those amounts. The results are shown in Table 2 and Table 3.

[Table 2]

|  | Rku | Charge amount | Fiber diameter | Activated platelet removal rate | Non-activated platelet removal rate | Activated platelet removal ratio | PDGF removal rate |
|---|---|---|---|---|---|---|---|
| Unit | - | mmol/g | μm | % | % | - | % |
| Example 1 | 0.5 | 0.0 | 30 | 82 | 12 | 6.8 | - |
| Example 2 | 1.1 | 0.0 | 33 | 64 | 12 | 5.3 | 2 |
| Example 3 | 5.8 | 0.0 | 37 | 42 | 12 | 3.5 | - |
| Example 4 | 12.2 | 0.0 | 38 | 38 | 13 | 2.9 | - |
| Example 5 | 15.6 | 0.0 | 38 | 25 | 10 | 2.5 | - |
| Comparative Example 1 | 18.4 | 0.0 | 38 | 12 | 11 | 1.1 | - |
| Example 6 | 0.5 | 0.5 | 32 | 86 | 13 | 6.6 | 70 |
| Example 7 | 1.1 | 0.5 | 33 | 68 | 11 | 6.2 | 73 |
| Example 8 | 6.2 | 0.5 | 37 | 43 | 11 | 3.9 | 77 |
| Example 9 | 12.8 | 0.5 | 38 | 40 | 13 | 3.1 | 79 |
| Example 10 | 15.8 | 0.5 | 38 | 26 | 10 | 2.6 | 74 |
| Example 11 | 1.0 | 0.1 | 34 | 67 | 11 | 6.1 | 12 |
| Example 12 | 1.0 | 0.9 | 34 | 66 | 12 | 5.5 | 92 |
| Example 13 | 1.1 | 1.4 | 33 | 65 | 11 | 5.9 | 70 |
| Example 14 | 1.1 | 2.0 | 38 | 66 | 11 | 6.0 | 42 |
| Example 15 | 2.8 | 0.5 | 36 | 64 | 10 | 6.4 | 64 |
| Example 16 | 1.0 | 2.6 | 36 | 56 | 11 | 5.1 | 27 |
| Example 17 | 1.2 | 3.5 | 40 | 40 | 11 | 3.6 | 13 |

[Table 3]

|  | VEGF removal rate | FGF removal rate |
|---|---|---|
| Unit | % | % |
| Example 1 | - | - |
| Example 2 | 3 | 2 |
| Example 3 | - | - |
| Example 4 | - | - |
| Example 5 | - | - |
| Comparative Example 1 | - | - |

(continued)

|  | VEGF removal rate | FGF removal rate |
| --- | --- | --- |
| Unit | % | % |
| Example 6 | 64 | 50 |
| Example 7 | 66 | 55 |
| Example 8 | 73 | 67 |
| Example 9 | 67 | 60 |
| Example 10 | 62 | 53 |
| Example 11 | 10 | 10 |
| Example 12 | 81 | 68 |
| Example 13 | 65 | 54 |
| Example 14 | 38 | 33 |
| Example 15 | 56 | 43 |
| Example 16 | 25 | 23 |
| Example 17 | 11 | 10 |

By the experimental results described above, the water-insoluble carrier of the present invention was shown to have excellent removal ability for activated platelets. It was also shown that the water-insoluble carrier also enables adsorption of growth factors in cases where it has a charged surface.

INDUSTRIAL APPLICABILITY

The water-insoluble carrier of the present invention is capable of removing activated platelets, and can be used for treatment of diseases for which suppression of thrombus formation can be expected to be therapeutically effective.

DESCRIPTION OF SYMBOLS

1    Area of analysis in one field of view in kurtosis measurement
2    Fraction of platelets bound to erythrocytes and the like, among CD41-positive cells
3    Platelet fraction of CD41-positive cells
4    Microparticle fraction of CD41-positive cells
1    Sampling length

**Claims**

1. A water-insoluble carrier which removes activated platelets, the carrier having a surface with a kurtosis of 0.1 to 16.0.

2. The water-insoluble carrier according to claim 1, wherein the surface has a charge.

3. The water-insoluble carrier according to claim 1 or 2, wherein the charge amount of the surface is 0.3 to 3.0 mmol per 1 g dry mass.

4. The water-insoluble carrier according to any one of claims 1 to 3, comprising an amino group on the surface.

5. The water-insoluble carrier according to any one of claims 1 to 4, which is a fiber or a particle.

6. The water-insoluble carrier according to claim 5, having a diameter of 1 to 100 μm.

7. The water-insoluble carrier according to any one of claims 1 to 6, comprising a polymer selected from the group consisting of polystyrene, polypropylene, polysulfone, polyether sulfone, and cellulose.

8. The water-insoluble carrier according to any one of claims 1 to 7, which removes a platelet-derived growth factor, vascular endothelial growth factor, and/or fibroblast growth factor.

9. A blood purification device, comprising the water-insoluble carrier according to any one of claims 1 to 8.

10. The blood purification device according to claim 9, which is a blood purification column.

11. The blood purification device according to claim 10, which is for suppression of thrombus formation.

Fig.1

Fig.2

## INTERNATIONAL SEARCH REPORT

| International application No. |
| --- |
| **PCT/JP2024/002358** |

| **A.** | **CLASSIFICATION OF SUBJECT MATTER** |
| --- | --- |

*A61M 1/36*(2006.01)i
FI: A61M1/36 119

According to International Patent Classification (IPC) or to both national classification and IPC

| **B.** | **FIELDS SEARCHED** |
| --- | --- |

Minimum documentation searched (classification system followed by classification symbols)

A61M1/36

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| **C.** | **DOCUMENTS CONSIDERED TO BE RELEVANT** |
| --- | --- |

| Category* | Citation of document, with indication, where appropriate, of the relevant passages | Relevant to claim No. |
| --- | --- | --- |
| X | WO 2021/066152 A1 (TORAY INDUSTRIES, INC.) 08 April 2021 (2021-04-08) paragraphs [0019]-[0024], [0060], [0087], [0090]-[0100] | 1-11 |
| A | JP 2011-30903 A (NIKKISO CO., LTD.) 17 February 2011 (2011-02-17) entire text, all drawings | 1 |
| A | JP 2020-163129 A (TORAY INDUSTRIES, INC.) 08 October 2020 (2020-10-08) entire text, all drawings | 1 |

☐ Further documents are listed in the continuation of Box C.　　☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **20 February 2024** | **26 March 2024** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**
Information on patent family members

International application No.

**PCT/JP2024/002358**

| Patent document cited in search report | | | Publication date (day/month/year) | Patent family member(s) | | | Publication date (day/month/year) |
|---|---|---|---|---|---|---|---|
| WO | 2021/066152 | A1 | 08 April 2021 | US | 2022/0362742 | A1 | |
| | | | | paragraphs [0029]-[0034], [0069], [0096], [0099]-[0109] | | | |
| | | | | EP | 4039327 | A1 | |
| | | | | CN | 114423470 | A | |
| | | | | KR | 10-2022-0074852 | A | |
| JP | 2011-30903 | A | 17 February 2011 | (Family: none) | | | |
| JP | 2020-163129 | A | 08 October 2020 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018225764 A
- WO 2019045031 A
- WO 2019049961 A
- WO 2019049962 A